# DEMANDE DE BREVET EUROPEEN

(11) **EP 2 805 684 A2**
(43) Date de publication de la demande: **26.11.2014**
(21) Numéro de dépôt: 14165570.4
(22) Date de dépôt: 23.04.2014
(51) Int. Cl.: A61B 19/00, A61L 2/00

(54) **Procédé et installation de nettoyage et de désinfection de dispositifs médicaux**

(30) Priorité: 22.05.2013 FR 1354561
(71) Demandeur: Air Liquide European Homecare Operations Services, 94250 Gentilly (FR)
(72) Inventeur: Chauvin, Eloise, 93300 Aubervilliers (FR)
(74) Mandataire: Pittis, Olivier

(57) **Abrégé**

L'invention porte sur un procédé de nettoyage et/ou de désinfection de tout ou partie d'un dispositif médical à nettoyer, dans lequel on met au moins une partie dudit dispositif médical en contact avec un fluide de nettoyage distribué par au moins une buse de distribution alimentée par du CO₂ à l'état liquide qui se transforme en particules de neige carbonique, qui sont projetées sur le dispositif médical à nettoyer. Préalablement à sa mise en contact avec le CO₂, au moins une partie dudit dispositif médical est soumise à un brossage mécanique et à une mise en contact avec un produit détergent/désinfectant dans le but d'améliorer l'efficacité du nettoyage. Installation de mise en oeuvre.

## Description

L'invention concerne un procédé et une installation de nettoyage et de désinfection applicable aux appareils et dispositifs médicaux, en particulier à ceux dits « fragiles » car comprenant des éléments ou composants électroniques fragiles, ou à ceux de structure ou architecture complexe donc difficiles à nettoyer par des techniques classiques.

Afin de garantir la propreté externe et la désinfection des appareils et dispositifs médicaux, il est habituel de leur faire subir régulièrement des opérations de nettoyage et désinfection consistant à nettoyer les salissures et à éliminer les micro-organismes présents sur leurs surfaces externes et/ou internes, voire sur certains organes ou autres éléments de ces dispositifs, de manière à prévenir les infections croisées et à limiter les niveaux de contamination des surfaces traitées.

Or, les méthodes classiques de nettoyage/désinfection des appareils et dispositifs médicaux sont souvent peu adaptées à ce type d'appareillage car elles impliquent des opérations manuelles, sont peu maîtrisées et peu reproductibles, et souvent nécessitent un pilotage délicat.

De plus, elles présentent une problématique globale de qualité-fiabilité, sécurité et productivité, et constituent par ailleurs un goulot d'étranglement dans la fluidité des opérations de reconditionnement des appareils et dispositifs médicaux, en particulier lorsque le processus de nettoyage est un procédé de type industriel, c'est-à-dire en continu sur chaîne de nettoyage par exemple.

En fait, beaucoup d'appareils et de dispositifs médicaux actuellement utilisés pour les soins en hôpital, à domicile ou ailleurs, ne sont pas compatibles, de par leur conception, avec les procédés de nettoyage/désinfection industriels car ils comportent des éléments fragiles, par exemple des composants électroniques, des matériaux absorbants ou autres, ou encore sont de configuration complexe, ce qui les rend non-immergeables et difficilement nettoyables par voie «humide» classique.

Il s'ensuit qu'il n'existe pas actuellement de procédé de nettoyage/désinfection applicable aux appareils et dispositifs médicaux fragiles, qui soit entièrement satisfaisant et utilisable au plan industriel.

Dans le cadre de la présente invention, les termes « dispositifs » et « appareils » médicaux sont considérés comme équivalents et sont utilisés indifféremment l'un de l'autre.

Le problème est donc de proposer un procédé et une installation de nettoyage/désinfection amélioré permettant d'industrialiser les opérations de nettoyage/désinfection externes des dispositifs et des appareils médicaux, y compris ceux comprenant des éléments, parties ou matériaux « fragiles » (composants électroniques...), en garantissant leur intégrité, et qui soit non seulement efficace dans l'élimination des micro-organismes (bactéries, virus...), salissures ou autres... mais aussi qui soit fiable, respecte les contraintes règlementaires et normatives et dont le coût de mise en oeuvre soit maîtrisé de sorte de ne pas affecter la rentabilité globale du processus de nettoyage/désinfection.

Une solution selon la présente invention est alors un procédé de nettoyage et/ou de désinfection de tout ou partie d'un dispositif médical à nettoyer, dans lequel on met au moins une partie dudit dispositif médical en contact avec un fluide de nettoyage distribué par au moins une buse de distribution alimentée par du CO₂ à l'état liquide, au moins une partie dudit CO₂ à l'état liquide se transformant en particules de neige carbonique, et au moins une partie desdites particules de neige carbonique étant projetées sur le dispositif médical à nettoyer, caractérisé en ce que, préalablement à sa mise en contact avec le CO₂, au moins une partie dudit dispositif médical est :
a) soumise à un brossage mécanique et
b) à une mise en contact avec un produit détergent/désinfectant.

Selon le cas, le procédé de l'invention peut comprendre l'une ou plusieurs des caractéristiques techniques suivantes :
- au moins une partie dudit CO₂ à l'état liquide se transformant en particules de neige carbonique lors de son passage dans ladite au moins une buse de distribution et/ou en amont et/ou en aval de ladite au moins une buse de distribution. En fait, le CO₂ est initialement utilisé à l'état liquide, c'est-à-dire que c'est du CO₂ liquide qui alimente le (ou les) pistolet et/ou la (ou les) buse portée(s) par le (ou les) pistolets de projection. Au moins une partie de ce CO₂ liquide, voire tout le CO₂ liquide, change cependant d'état et passe de l'état liquide à l'état solide, lors de son passage dans le (ou les) pistolet de projection et/ou dans la (ou les) buse pour former des microparticules de glace, c'est-à-dire des particules de neige carbonique, qui, par projection, peuvent atteindre une vitesse supersonique en sortie de buse(s), typiquement supérieure à MACH 1, par exemple de l'ordre de MACH 1,3. Ce sont notamment ces particules de glace de CO₂ qui sont directement appliquées sur les surfaces du dispositif médical et qui permettent alors son nettoyage. Les particules de neige carbonique se subliment ensuite en CO₂ gazeux, notamment lors de leur contact avec la surface du dispositif On obtient au final un effet mécanique, thermique et de souffle du ou des jets de CO₂ solide et/ou gazeux dispensés par la ou les buses de projection permettant ainsi d'obtenir le décapage et nettoyage de surface désiré. En d'autres termes, au moins une partie desdites particules de neige carbonique sont projetées sur le dispositif médical à nettoyer permettant ainsi d'obtenir un décapage et/ou un nettoyage de surface désiré.
- au moins une buse de distribution est portée par au moins un pistolet de projection, au moins une partie du CO₂ à l'état liquide se transformant en particules de neige carbonique, lors de son passage dans ledit au moins un pistolet de projection et dans ladite au moins une buse de distribution.
- au moins une partie des particules de neige carbonique se subliment en CO₂ gazeux, après leur sortie de ladite au moins une buse de distribution.
- un décapage ou nettoyage de surface du dispositif médical à nettoyer est obtenu par effet mécanique, thermique et de souffle du CO₂ solide et/ou gazeux dispensé par la ou les buses de projection.
- la mise en contact avec le produit détergent/désinfectant est opérée par pulvérisation préalable de produit détergent/désinfectant.
- le produit détergent et/ou désinfectant comprend du chlorure de didécyldiméthylammonium, du chlorhydrate de polyhexaméthylène biguanide et des agents détergents, filmant et moussant.
- le brossage mécanique est opéré au moyen d'une ou plusieurs brosses rotatives.
- le dispositif médical comprend au moins un composant fragile, en particulier un (ou plusieurs) composant électronique ou un matériau absorbant.
- le dispositif médical est choisi parmi les appareils d'assistance respiratoire (par exemple les appareils de traitement de l'apnée du sommeil, les ventilateurs, les dispositifs portables d'oxygène liquide, les concentrateurs d'oxygène, les aspirateurs trachéaux, les générateurs d'aérosols...) et les appareils de perfusion, de nutrition, de traitement du diabète, ou tout autre appareil médical.
- au moins une partie dudit dispositif médical est mise en contact avec un fluide de désinfection finale subséquemment, à la mise en contact avec le CO₂, c'est-à-dire que fluide de désinfection finale est appliqué après le CO₂.
- le fluide de désinfection finale comprend un (ou plusieurs) biocide vaporisé.
- le fluide de désinfection finale comprend du peroxyde d'hydrogène (H₂O₂) ou de l'acide peracétique.
- on opère un nettoyage et/ou une désinfection d'au moins une partie de la surface externe et/ou de la surface interne du dispositif.
- on opère un nettoyage et/ou une désinfection d'au moins un organe ou composant situé à l'intérieur ou à l'extérieur du dispositif, par exemple un organe de réglage ou un circuit de ventilation.
- le CO₂ est distribué sous forme d'un ou plusieurs jets de CO₂.
- le CO₂ est distribué à une pression d'au moins 1 bar, de préférence entre 1,5 et 25 bar, de préférence encore entre 2 et 15 bar, typiquement entre 2 et 6 bar.
- le CO₂ est distribué à vitesse supersonique, par exemple de l'ordre MACH 1,3.
- le CO₂ est à une température inférieure à -20°C.
- le jet de CO₂ est projeté sur la surface à traiter par un flux d'air comprimé, de préférence un flux d'air concentrique gainant le jet de CO₂.
- le CO₂ est distribué par une ou plusieurs buses de distribution. Les buses peuvent avoir un orifice de sortie de forme circulaire ou de fente aplatie.

Par ailleurs, l'invention concerne aussi une installation de nettoyage et/ou de désinfection de dispositif médical susceptible de mettre en oeuvre le procédé de l'invention, c'est-à-dire le procédé de nettoyage décrit ci-dessus, comprenant :
- une installation d'alimentation en CO₂ liquide comprenant un réservoir contenant du CO₂ liquide, un dispositif de compression ou de pompage et au moins une canalisation d'acheminement de CO₂ permettant d'acheminer du CO₂ liquide du réservoir au dispositif de compression ou de pompage, puis dudit dispositif de compression ou de pompage à une unité de traitement cryogénique,
- une unité de traitement cryogénique comprenant une ou plusieurs buses de distribution de jet de CO₂, alimentées en CO₂ liquide par ladite au moins une canalisation d'acheminement de CO₂,
- une première unité de pulvérisation comprenant un réservoir de liquide détergent/désinfectant alimentant fluidiquement un ou plusieurs moyens de pulvérisation permettant de pulvériser au moins une partie du détergent/désinfectant provenant dudit réservoir à liquide détergent/désinfectant, et
- une unité de brossage comprenant une ou plusieurs brosses.

Selon le cas, l'installation de l'invention peut comprendre l'une ou plusieurs des caractéristiques techniques suivantes :
- le ou les moyens de pulvérisation comprennent une ou plusieurs buses de pulvérisation.
- l'unité de brossage comprenant une ou plusieurs brosses rotatives.
- elle comprend en outre une deuxième unité de pulvérisation comprenant un réservoir de biocide alimentant fluidiquement un générateur de nébulisât permettant de nébuliser au moins une partie du biocide provenant dudit réservoir à biocide.
- elle comprend en outre un dispositif convoyeur permettant d'acheminer au moins un dispositif médical à nettoyer au sein d'au moins l'unité de traitement cryogénique.
- elle comprend en outre une unité de compression d'air permettant d'alimenter la ou les buses avec de l'air comprimé.
- elle comprend en outre une unité de pilotage comprenant un coffret de commande destiné à réguler la distribution de CO₂ liquide et d'air comprimé à ladite ou auxdites buses.
- au moins une première unité de pulvérisation et/ou au moins une unité de brossage sont agencées en amont de l'unité de traitement cryogénique, et/ou au moins une deuxième unité de pulvérisation est agencée en aval de l'unité de traitement cryogénique.
- la ou les buses sont situées dans une enceinte fermée, en particulier un tunnel muni de portes d'entrée et de sortie.
- la ou les buses sont portées par une poutre ou un bras robotisé.
- la deuxième unité de pulvérisation comprend une enceinte fermée au sein de laquelle le générateur de nébulisât est apte à créer un nébulisât de biocide.

L'invention va maintenant être mieux comprise grâce à la description détaillée suivante faite en référence aux Figures annexées parmi lesquelles :
- la Figure 1 schématise un mode de réalisation d'une installation de mise en oeuvre du procédé de nettoyage et de désinfection selon la présente invention,
- les Figures 2 et 3 illustrent l'action du jet de CO₂ sur la surface à nettoyer,
- la Figure 4 schématise un mode de réalisation d'une installation d'alimentation en CO₂ de l'unité de nettoyage de la Figure 1 ;
- la Figure 5 schématise un mode de réalisation de l'unité de nettoyage de la Figure 1 ; et
- la Figure 6 représente un exemple de cycle de désinfection au sein de l'unité de désinfection de la Figure 1.

Dans le cadre de la présente invention, il est proposé un procédé de nettoyage et de désinfection des dispositifs ou appareils médicaux électroniques, par exemples les appareils d'assistance respiratoire, circuit internes des ventilateurs, ventilateurs eux-mêmes, portables d'oxygène liquide, concentrateurs d'oxygène, manodétendeurs, aspirateurs trachéaux, générateurs d'aérosols, pompes à insuline, pompes à perfusion..., comprenant notamment une étape de nettoyage de la surface externe à nettoyer par projection de CO₂, de préférence opérée au sein d'une unité de traitement cryogénique, comme expliqué ci-après.

Avantageusement, le procédé comprend également les étapes suivantes :
i) pulvérisation de produits détergent/désinfectant, de préférence au sein d'une première unité de pulvérisation située en amont de l'unité de traitement cryogénique,
ii) brossage par dispositif mécanique, tel qu'une ou plusieurs brosses ou analogues, de préférence au sein d'une unité de brossage située en amont de l'unité de traitement cryogénique, et
iii) optionnellement, désinfection finale de la surface au moyen d'un produit biocide, de préférence au sein d'une deuxième unité de pulvérisation finale située en aval de l'unité de traitement cryogénique. Cette seconde étape de désinfection finale de la ou des surfaces nettoyées devant encore être désinfectées, se fait par dispensation par voie aérienne, typiquement par vaporisation sur ladite/lesdites surfaces, d'un biocide de type peroxyde d'hydrogène (H₂O₂) ou acide peracétique.

La projection de CO₂ se déroule donc après les étapes i) et ii) mais avant l'étape optionnelle iii), lorsque celle-ci est mise en oeuvr, c'est-à-dire entre la première unité de pulvérisation et/ou l'unité de brossage, et par ailleurs la deuxième unité de pulvérisation finale.

L'efficacité de l'étape de projection de CO₂ est conditionnée par l'efficacité des étapes i) et ii) précédentes.

Plus généralement, les surfaces concernées par le procédé de nettoyage et de désinfection de l'invention sont les surfaces externes, c'est-à-dire situées du côté extérieur du dispositif à nettoyer, et/ou les surfaces internes, c'est-à-dire situées à l'intérieur du dispositif à nettoyer, y compris les surfaces de certains composants ou organes situés dans ou hors de l'appareil à nettoyer et désinfecter.

Ainsi, le procédé de l'invention a montré une efficacité dans la désinfection du circuit d'insufflation d'un appareil d'assistance respiratoire.

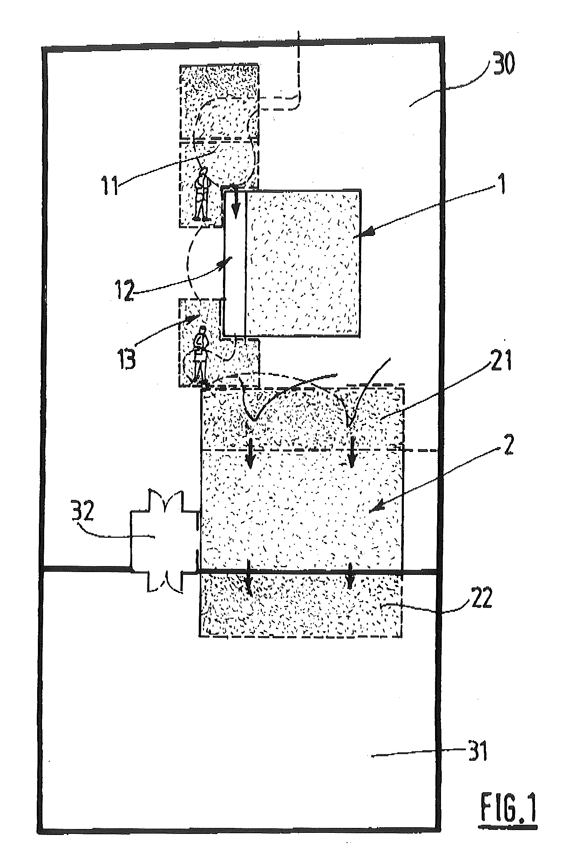

Les étapes du procédé sont préférentiellement mises en oeuvre au sein d'unités de traitement spécifiques comme illustré en Figure 1.

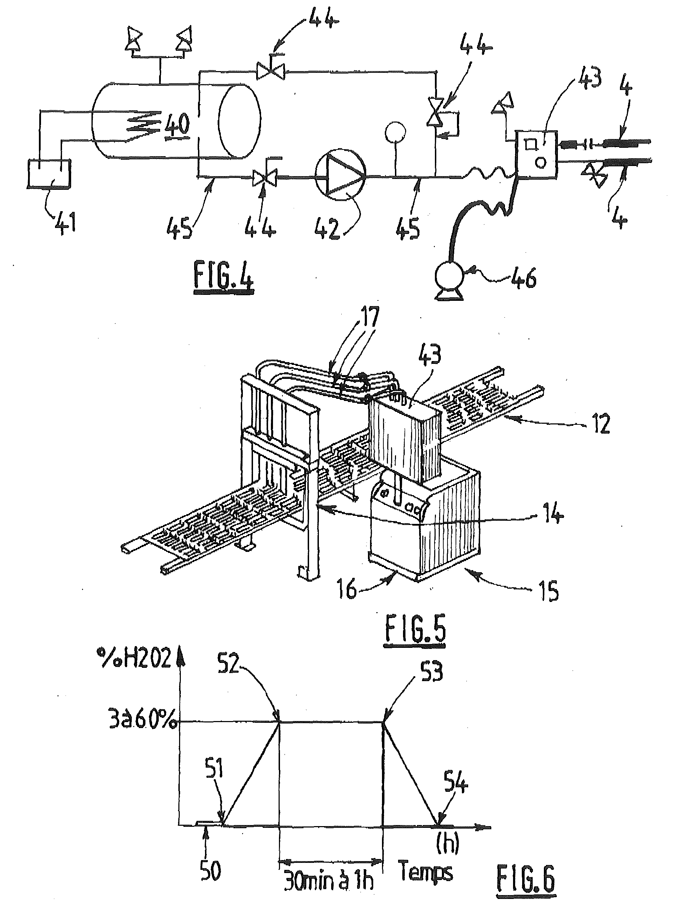

Ainsi, comme on peut le voir, l'étape de nettoyage par projection de CO₂ se fait au sein d'une première unité de traitement cryogénique 1, encore appelée unité de nettoyage cryogénique, au sein de laquelle sont agencées une ou plusieurs buses 4 de projection de CO₂. Ces buses 4 sont alimentées en CO₂ liquide cryogénique par exemple par l'installation schématisée en Figure 4 et qui sera détaillée ci-après.

Ensuite, l'étape de désinfection finale par pulvérisation ou vaporisation de biocide, par exemple de type peroxyde d'hydrogène ou d'acide peracétique se fait au sein d'une seconde unité de pulvérisation 2, encore appelée unité de désinfection finale, laquelle est située en aval de l'unité de traitement cryogénique 1 délivrant le ou les jets de CO₂.

En fait, les dispositifs à nettoyer et désinfecter arrivent dans une zone de réception et de chargement 11 située en amont de l'unité de nettoyage cryogénique 1 proprement dite, puis traversent ladite unité de nettoyage cryogénique 1 par exemple sur un tapis de convoyage 12 ou analogue, avant d'être récupérés, une fois nettoyés, dans une zone de réception 13 située en aval de l'unité de nettoyage cryogénique 1.

Les dispositifs nettoyés sont alors transférés à une zone de transfert et chargement 21 située en amont de l'unité de désinfection finale 2 proprement dite, puis sont désinfectés par pulvérisation de biocide dans ladite unité de désinfection finale 2, avant d'être récupérés, une fois nettoyés et désinfectés, dans une zone de déchargement 22 située en aval de l'unité de désinfection finale 2.

De préférence, l'unité de nettoyage cryogénique 1 et l'unité de désinfection finale 2, ainsi qu'avantageusement les zones 11, 13, 21 situées en amont ou entre ces unités 1, 2, sont préférentiellement situées dans un premier local, alors que la zone de déchargement 22 située en aval de l'unité de désinfection 2 finale, est préférentiellement agencée dans un second local 31, adjacent au premier local 30 et communiquant via un sas 32.

De préférence, le second local 31 est de type « salle propre », c'est-à-dire un local au sein duquel le niveau de propreté est assez élevé et les conditions d'hygiène maitrisées, et où les appareils pourront donc subir des opérations de finition avant leur ré-emballage et leur expédition.

L'étape i) de pulvérisation de produits détergent/désinfectant et/ l'étape ii) de brossage par dispositif mécanique, tel qu'une ou plusieurs brosses ou analogues, opérées préalablement à l'étape de projection de CO₂, sont mises en oeuvre dans des installations spécifiques non illustrées sur les Figures.

Une fois ces étapes préalables i) et ii) mises en oeuvre pour opérer un pré-nettoyage ou nettoyage « grossier » de l'appareil à nettoyer, on opère l'étape de nettoyage par CO₂ ou nettoyage « précis » de l'appareil, puis sa désinfection finale comme illustré en Figure 1 à 3.

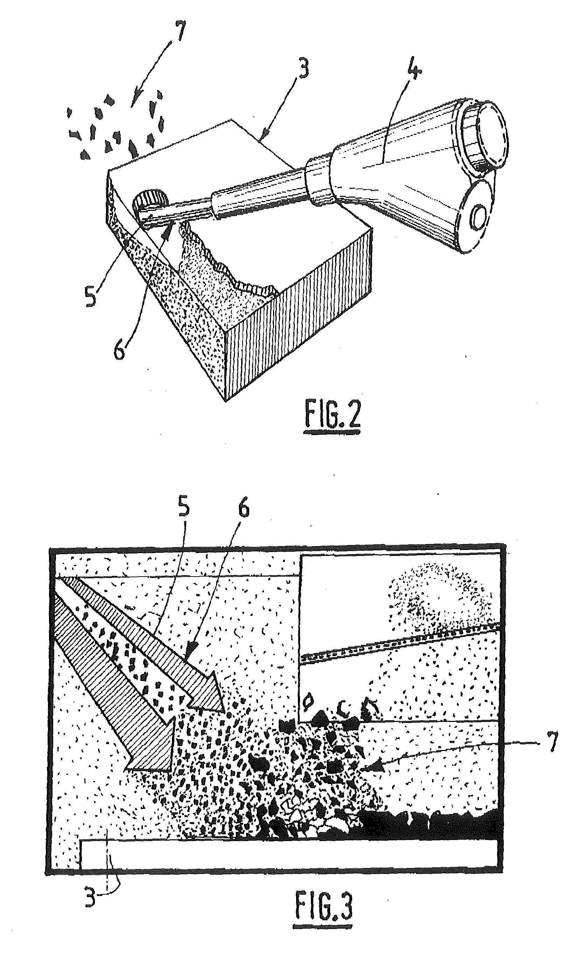

Plus précisément, dans l'unité de traitement 1 cryogénique, comme illustré en Figures 2 et 3, chaque jet de CO₂ 5 est projeté sur la surface à traiter 3 où il va agir en éliminant les salissures 7 ou analogues qui s'y trouvent, y compris dans les endroits difficiles lorsque l'architecture du dispositif est complexe. De préférence, chaque jet de CO₂ 5 est gainé par un flux d'air comprimé concentrique 6 délivré par la buse 4. A l'endroit où le (ou les) jet de CO₂ 5 impacte la surface à traiter 3, celui-ci va décoller les salissures 7, souillures ou analogues se trouvant sur ladite surface, du fait de l'effet thermique, de l'effet mécanique et de l'effet de souffle du et de CO₂.

L'effet thermique résulte de la température cryogénique du CO₂, c'est-à-dire par exemple à une température de l'ordre de -80°C. Par ailleurs, l'effet mécanique résulte de la force d'impact de chaque jet 5, 6 sur la surface 3 et varie en fonction de la vitesse du jet, du débit, du type de buse de distribution équipant le pistolet ou analogue de projection. En outre, l'effet de souffle est engendré par la sublimation du CO₂ à l'impact, qui occupe alors instantanément environ 700 fois son volume.

En fait, le CO₂ liquide alimente un ou des pistolets de projection qui sont chacun munis d'une buse de projection. Au moins une partie de ce CO₂ liquide, voire tout le CO₂ liquide, change d'état et passe de l'état liquide à l'état solide, lors de son passage dans chaque pistolet de projection et/ou dans la (ou les) buse pour former des particules solides de neige carbonique, qui sont alors projetées à vitesse supersonique vers la surface à nettoyer de l'appareil médical. Ces particules de glace carbonique permettent alors le nettoyage de la surface impactée. Tout ou partie des particules de neige carbonique se sublime ensuite en CO₂ gazeux, lors de leur contact avec la surface du dispositif, et on obtient au final un effet mécanique, thermique et de souffle du jet de CO₂ solide et/ou gazeux dispensé par la ou les buses de projection permettant ainsi d'obtenir le décapage de surface désiré.

Un tel jet de CO₂ sous plusieurs états est très efficace et présente l'avantage de ne pas être « humide » et d'être neutre, donc ne pas risquer d'abimer les éléments et autres composants fragiles, notamment les composants électroniques (cartes électroniques, mémoires...) des dispositifs médicaux soumis au procédé de l'invention.

Un mode de réalisation d'une installation d'alimentation en CO₂ de la première unité de traitement 1 est illustré en Figure 4.

Elle comprend un réservoir 40 de CO₂ liquide à basse pression contenant par exemple du CO₂ à l'état liquide à une température de -20°C environ et à une pression de l'ordre de 20 bar absolus. La température du CO₂ stocké au sein du réservoir 40 est contrôlée grâce à un groupe frigorifique 41. Le CO₂ liquide est soutiré du réservoir 40 par une ou plusieurs canalisations 45 avant d'être comprimé par une pompe de remontée en pression 42 puis envoyé vers la ou les buses de distribution 4 de l'unité de traitement cryogénique 1. Le contrôle de la distribution du CO₂ se fait au moyen d'un coffret de commande et de distribution 43 agencé entre la pompe 42 et les buses 4. Des vannes 44 servent à réguler la circulation du CO₂ au travers de la ou des canalisations 45. De préférence, les canalisations sont isolées pour éviter que le CO₂ ne se vaporise en se réchauffant au sein de celles-ci pendant son transfert.

Par ailleurs, un mode de réalisation de l'unité de traitement cryogénique 1 est illustré en Figure 5. Les dispositifs médicaux à nettoyer y sont convoyés par un dispositif convoyeur 12, par exemple un convoyer à rouleaux ou un tapis roulant, et sont soumis à des jets de CO₂ distribués par un ensemble de buses 4 de distribution alimentées en CO₂ liquide issu par exemple de l'installation d'alimentation en CO₂ de la Figure 4.

Par ailleurs, une unité de compression 46 permet d'alimenter également les buses 4 en air comprimé de manière permettre de gainer les jets de CO₂ d'un jet concentrique d'air comprimé, ce qui permet de garantir une bonne efficacité de décapage et de nettoyage des surfaces traitées.

Préférentiellement, l'air comprimé est délivré à une pression de l'ordre de 2 à 15 bar abs, typiquement de l'ordre de 5 bar abs, et à un débit de 90 à 400 m³/h. Avantageusement, l'air comprimé est préalablement filtré et déshuilé.

Les flux de gaz, c'est-à-dire le CO₂ et l'air comprimé, sont convoyés par des canalisations 17 reliées à l'ensemble de buses 4, typiquement un ensemble comprenant 4 à 6 rangées de buses 4 réparties de manière à traiter l'intégralité de la surface à nettoyer au moyen des jets de CO₂ délivrés par lesdites buses 4.

Idéalement, les buses 4 sont agencées pour que les jets de CO₂ viennent nettoyer toutes les faces et/ou surfaces externes de l'appareil médical. Ainsi, des buses 4 seront agencées non seulement au dessus du convoyeur 12 à rouleaux mais aussi sur les côtés de celui-ci et en-dessous dudit convoyeur 12 de manière à ce que les jets de CO₂ puissent venir frapper les surfaces à nettoyer, non seulement par le dessus mais aussi latéralement et par le dessous.

Alternativement, on peut prévoir également un dispositif de manipulation, tel un bras robotisé manipulateur, permettant de déplacer l'appareil médical à nettoyer pour changer son orientation et permettre une exposition de toutes ses surfaces aux jets issus des buses 4.

La distribution des flux et plus généralement le fonctionnement de l'installation est commandé par une unité de pilotage 16 comprenant le coffret de commande 43 qui coopère avec l'unité de pilotage 16.

A titre d'exemple, on peut utiliser des buses 4 ayant un orifice de sortie aplati d'environ 40 mm de largeur de manière à avoir une largeur du flux de décapage du même ordre de grandeur. Bien entendu, on peut également utiliser des buses présentant une largeur différente, c'est-à-dire supérieure ou inférieure à 40 mm, et/ou des buses ayant une forme d'orifice de sortie différente, par exemple de forme circulaire, oblongue, ovale, polygonale ou autre, et ce, notamment en fonction de l'opération à effectuer et du type d'appareils à nettoyer.

La vitesse d'éjection peut être supersonique, par exemple de l'ordre de MACH 1,3 et la pression d'utilisation des jets de CO₂ de l'ordre de 3 bar abs, ce qui conduit à un jet de vitesse théorique de l'ordre de 350 mm/sec. Bien entendu, là encore, on peut mettre en oeuvre des pressions et vitesses supérieures ou inférieures à celles susmentionnées, en fonction de l'opération à effectuer et du type d'appareils à nettoyer. Pour les valeurs suscitées et le type de buse susmentionnés, on obtient une consommation de l'ordre de 100 Nm³/h d'air et d'environ 30 kg/h de CO₂.

Pour un nettoyage optimisé, il est recommandé d'orienter le jet CO₂ cryogénique à une certaine distance et sous un certain angle, définis par rapport à la surface à traiter. La trajectoire à effectuer et la durée d'application du jet sont également des facteurs importants. Ces paramètres d'optimisation sont à la portée de tout homme du métier et peuvent être obtenus aisément via de simples réglages par tâtonnements et des essais de routine. En fait, chaque dispositif médical dispose de ses propres spécificités et de tels réglages devront être effectués au cas par cas s'il l'on souhaite optimiser le procédé.

Préférentiellement, compte tenu de l'utilisation de CO₂, l'ensemble des buses 4 et au moins une partie du convoyeur 12 sont préférentiellement agencés dans une cabine ou une enceinte 15 insonorisée et ventilée, par exemple un tunnel. Un tel tunnel 15 est avantageusement équipée de portes d'entrée et sortie automatisées, situées en amont et en aval des buses en considérant le sens de progression des appareils sur le convoyer 12, de manière à s'ouvrir lorsqu'un appareil à nettoyer se présente et à se refermer après son entrée dans l'enceinte 15, et de manière similaire à s'ouvrir puis se refermer au même de sa sortie, de manière limiter les sorties de CO₂ de l'enceinte et à limiter les entrées de chaleur au sein de l'enceinte.

Au sein de l'unité de traitement 1 cryogénique, la distribution des jets de CO₂ peut également se faire, selon un autre mode de réalisation (non illustré), au moyen d'un robot à un ou plusieurs bras articulés équipés des buses 4, par exemple un (ou plusieurs) bras robotisé à 6 axes avec système de préhension pour tenir un pistolet équipé d'une ou plusieurs buses 4 de distribution des jets de CO₂ ou, de manière alternative, un bras robotisé équipé d'une potence fixe pour suspension du bras robotisé au niveau de la zone de travail. De tels robots sont notamment disponibles auprès de la société FANUC.

Par ailleurs, l'installation peut également être équipée d'une (ou plusieurs) caméra 2D ou 3D par exemple à laser et système de lecture de code barre, de reconnaissance RFID ou analogue pour permettre une reconnaissance du dispositif médical à nettoyer et son recentrage ou son orientation correcte sur le convoyeur 12, voire la mise en oeuvre d'un programme de nettoyage spécifique par activation de buses données.

L'installation peut aussi comprendre un système de détection et d'ajustement des rangées de buses 4, par exemple un système doté de cellules optiques, afin d'ajuster la distance du jet de CO₂ cryogénique en fonction de la configuration du dispositif à nettoyer.

De même, on peut prévoir un système de commande et de programmation du robot pour opérer un apprentissage de trajectoire et par ailleurs un système de sécurité pour l'opérateur avec système de départ ou d'arrêt d'urgence.

En définitive, le cycle de nettoyage d'un appareil médical au sein du tunnel 15 de l'unité de traitement 1 cryogénique peut se faire selon les étapes suivantes :
a) l'opérateur dépose le (ou les) dispositif(s) à nettoyer sur la zone de réception 11 située en entrée du tunnel 15, le (ou les) dispositif(s) pouvant avoir fait l'objet d'un pré-nettoyage (étape (i))ou d'un brossage préalable (étape (ii)) comme expliqué ci-avant, b) le dispositif à nettoyer est acheminé dans le tunnel 15 par convoyeur à rouleaux 12 ou tout autre type de convoyeur adapté,
b) détection du dispositif à nettoyer et transfert vers les rangées de buses 4,
c) ajustement automatique des rangées de buses 4 suivant les dimensions du dispositif à nettoyer notamment pour permettre une distance optimale de projection de CO₂ cryogénique sur les surfaces à nettoyer,
d) nettoyage des 6 faces de l'objet par les rangées de buse 4, et
e) évacuation du dispositif nettoyé en sortie de tunnel 15 vers la zone de réception 13.

Les appareils médicaux ainsi nettoyés peuvent ensuite être traités dans la zone de désinfection finale, c'est-à-dire vers l'unité de désinfection 2 finale.

Au sein de l'unité de désinfection finale 2, les appareils sont soumis à une désinfection finale des surfaces externes par voie aérienne en utilisant un agent biocide vaporisé tel que du peroxyde d'hydrogène (H₂O₂). Bien entendu, d'autres agents biocides peuvent également convenir.

Pour ce faire, au sein de l'unité de désinfection finale 2 qui comprend elle-aussi une enceinte fermée, on génère un brouillard par nébulisation du biocide au moyen d'un générateur de nébulisât à atomiseur.

Le cycle de désinfection d'un appareil médical au sein de l'unité de désinfection finale 2 peut se faire selon les étapes suivantes et est schématisé en Figure 6 :
a) chargement de (ou des) l'appareil à désinfecter via une zone de chargement 21, par exemple une porte donnant accès à l'intérieur de l'enceinte où s'effectue le traitement par biocide. Le ou les appareils peuvent par exemple être placés sur des chariots mobiles qui sont introduits par un opérateur dans l'enceinte de traitement.
b) fermeture étanche (en 50) des portes de l'enceinte.
c) lancement d'un cycle automatique de traitement par nébulisation (en 51), de préférence selon un programme préenregistré, commandable par exemple par activation d'un bouton marche/arrêt ou analogue.
d) injection et diffusion du produit biocide et saturation de l'atmosphère de l'enceinte avec ledit produit (fin de nébulisation : 52). Par exemple, le nébulisat ou brouillard de biocide peut comprendre de 3 à 60% (% en volume) de biocide, typiquement H₂O₂, par exemple de l'ordre de 30% de biocide.
e) phase de contact biocide/appareil pendant le temps nécessaire pour atteindre le niveau d'efficacité attendu du biocide (entre 52 et 53). Par exemple, un temps de contact de 30 minutes à 1 heure.
f) phase d'aération ou extraction destinée à éliminer le produit résiduel (entre 53 et 54).
g) extraction (à partir de 54) des appareils désinfectés via une zone de déchargement 22.

Le biocide est avantageusement du peroxyde d'hydrogène (H₂O₂) car ce produit ne laisse aucun résidu sur les appareils et a montré son efficacité dans l'élimination des germes, notamment bactériens.

Toutefois, afin d'augmenter le spectre d'activité de la composition biocide, on peut aussi utiliser un mélange de plusieurs biocides ou une utilisation successives de plusieurs biocides vaporisés l'un après l'autre dans l'enceinte, par exemple du peroxyde d'hydrogène et de l'acide peracétique. Utiliser du peroxyde d'hydrogène pur sans résidu présente également l'avantage de permettre une désinfection interne des ventilateurs et des circuits d'insufflation en minimisant le risque de relarguage chimique. Le biocide peut être stocké dans un réservoir situé dans ou hors du local 31.

Par ailleurs, on prévoit avantageusement un système de monitorage du taux de biocide résiduel à l'extérieur de l'enceinte de nébulisation et/ou une sonde à biocide à l'intérieur de ladite enceinte.

Il est à noter que le nettoyage et la désinfection des parties, éléments, composants et surfaces internes des appareils se déroulent de manière similaire à ce qui a été présenté ci-dessus.

L'installation et le procédé de l'invention se sont montrés particulièrement efficaces dans le nettoyage et la désinfection d'appareils médicaux, notamment de ventilateurs comprenant des composants électroniques fragiles et sensibles à l'humidité, de circuits de ventilation et d'autres équipements analogues.

### Exemple

Afin de vérifier l'efficacité du procédé de nettoyage et de désinfection de l'invention, plusieurs essais de nettoyage/désinfection d'appareils médicaux ont été réalisés comme expliqué ci-après.

Un appareil médical a été volontairement souillé avec des salissures colorées (de manière à pouvoir opérer un test visuel) déposées au pinceau sur la surface externe de l'appareil, y compris dans des zones d'accès difficile (poignée...).

Les buses de distribution de CO₂ testées étaient des buses :
- courte de 50 mm MACH 1 .8 délivrant une pression de 6 bar à un débit de 150 Nm3/h, de la société ALTERCLEAN,
- longue de 40 mm MACH 1 .3 délivrant une pression de 3 bar à un débit de 100 Nm3/h, de la société ALTERCLEAN,
- courte de 40 mm MACH 1 .3 délivrant une pression de 3 bar à un débit de 100 Nm3/h, de la société ALTERCLEAN,
- longue de 18 mm délivrant une pression de 6 bar à un débit de 120 Nm3/h de la société MYCON,
- courte de 30 mm délivrant une pression de 6 bar à un débit de 150 Nm3/h de la société MYCON.

Le débit de CO₂ était de 15 à 60 kg/h, et la pression d'air de gainage entre 3 et 6 bar. La distance buse/surface est située entre 15 et 20 cm.

L'étape de nettoyage par pulvérisation de CO₂ cryogénique peut être précédée, selon les essais, par une étape de brossage et/ou de nettoyage/désinfection par un produit détergent/désinfectant. Dans ce cas, les brosses utilisées sont des brosses plates rotatives (300 à 700 tr/min) et le produit détergent de nettoyage/désinfection utilisé est commercialisé par la société ANIOS sous la référence SurfaSafe Anios, lequel doit être appliqué pendant 5 minutes (temps de contact) sans séchage. Le produit Surfasafe est une solution limpide incolore, contenant du chlorure de didécyldiméthylammonium, du chlorhydrate de polyhexaméthylène biguanide et des agents détergents, filmant et moussant.

A titre comparatif, des essais ont également été opérés à l'aide de lingettes imprégnées de produit de nettoyage disponibles dans le commerce.

L'installation utilisée pour distribuer le CO₂ est analogue à celle représentée sur les Figures, comme expliqué ci-dessus.

L'efficacité du nettoyage est évaluée par examen visuel des surfaces traitées et constat ou non de la présence de résidus : poussières, salissures...

Les essais et résultats obtenus sont résumés dans le Tableau suivant.

**Tableau**

| Essai n° | Lingette de nettoyage | Produit détergent/désinfectant | Brossage | Projection de CO₂ | Résultats |
|---|---|---|---|---|---|
| A | / | / | / | oui | + |
| B | / | oui | / | oui | ++ |
| C | / | oui | oui | oui | +++ |
| D | / | oui | oui | / | - |
| E | Oui | / | / | / | - |

| | | | | | |
|---|---|---|---|---|---|
| + : nettoyage satisfaisant (i.e. > 60% d'appareils totalement nettoyés) ++ / +++: nettoyage très satisfaisant / quasi parfait - / -- : nettoyage insatisfaisant / très insuffisant | | | | | |

Comme on le voit, lorsque l'on n'opère pas d'étape de projection de CO₂, le nettoyage des appareils est insuffisant (Essai D), voire très insuffisant (Essai E), puisque les appareils sont toujours recouverts de salissures sur plus de 40% de leur surface externe, même lorsque ces salissures sont des salissures moyennement incrustées et/ou de simples poussières.

Par contre, dès lors qu'une étape de projection de CO₂ est mise en oeuvre (Essai A à C), le nettoyage des appareils devient plus efficace et ce, quelle que soit la buse utilisée et les conditions de traitement puisque les salissures peuvent être éliminées sur plus de 60% des surfaces traitées.

Toutefois, on comprend que cela n'est pas suffisant est qu'il convient, s'agissant d'appareils médicaux de pouvoir éliminer les salissures sur plus de 80 à 90%, voire même plus. Pour ce faire, selon l'invention, on a fait précéder l'étape de projection de CO₂ d'étapes supplémentaires de pré-nettoyage par brossage et/ou pulvérisation de détergent (Essai B et C).

Comme on le voit, les meilleurs résultats sont obtenus pour l'essai C conforme à l'invention, dans le cadre duquel les appareils ont été nettoyés par pulvérisation de détergent, brossage et enfin projection de particules de CO₂. En effet, c'est cet Essai C qui permet d'atteindre un niveau de propreté suffisant, c'est-à-dire d'au moins 80 à 90% des surfaces traitées.

Une telle combinaison d'étapes selon l'invention est particulièrement utile et recommandée, voire quasi indispensable, pour traiter des appareils très sales, c'est-à-dire des appareils particulièrement souillés et/ou comprenant des salissures plus fortement adhérentes.

En outre, afin de garantir un niveau d'hygiène amélioré, on peut également prévoir une ou plusieurs étapes post projection de CO₂, de nettoyage final avec un produit de désinfection du type H₂O₂.

Dans tous les cas, les essais ci-dessus montrent l'efficacité du procédé de l'invention qui combine les effets des étapes i) à iii) susmentionnées.

## Revendications

1. Procédé de nettoyage et/ou de désinfection de tout ou partie d'un dispositif médical à nettoyer, dans lequel on met au moins une partie dudit dispositif médical en contact avec un fluide de nettoyage distribué par au moins une buse de distribution alimentée par du CO₂ à l'état liquide, au moins une partie dudit CO₂ à l'état liquide se transformant en particules de neige carbonique, et au moins une partie desdites particules de neige carbonique étant projetées sur le dispositif médical à nettoyer,
**caractérisé en ce que**, préalablement à sa mise en contact avec le CO₂, au moins une partie dudit dispositif médical est :
a) soumise à un brossage mécanique et
b) à une mise en contact avec un produit détergent/désinfectant.

2. Procédé selon la revendication précédente, **caractérisé en ce qu'**au moins une buse de distribution est portée par au moins un pistolet de projection, au moins une partie du CO2 à l'état liquide se transformant en particules de neige carbonique, lors de son passage dans ledit au moins un pistolet de projection et dans ladite au moins une buse de distribution.

3. Procédé selon l'une des revendications précédentes, **caractérisé en ce qu'**au moins une partie des particules de neige carbonique se subliment en CO₂ gazeux, après leur sortie de ladite au moins une buse de distribution.

4. Procédé selon l'une des revendications précédentes, **caractérisé en ce que** le jet de CO₂ est projeté sur la surface à traiter par un flux d'air comprimé, de préférence un flux d'air concentrique gainant le jet de CO₂.

5. Procédé selon l'une des revendications précédentes, **caractérisé en ce qu'**au moins une partie dudit dispositif médical est mise en contact avec un fluide de désinfection finale subséquemment à la mise en contact avec le CO₂.

6. Procédé selon l'une des revendications précédentes, **caractérisé en ce que** le fluide de désinfection finale est un biocide vaporisé.

7. Procédé selon l'une des revendications précédentes, **caractérisé en ce que** le fluide de désinfection finale comprend du peroxyde d'hydrogène (H₂O₂) et/ou de l'acide peracétique.

8. Installation de nettoyage et/ou de désinfection de dispositif médical susceptible de mettre en oeuvre le procédé selon l'une des revendications précédentes, comprenant :
- une installation d'alimentation en CO₂ liquide comprenant un réservoir (40) contenant du CO₂ liquide, un dispositif de compression ou de pompage (42) et au moins une canalisation d'acheminement de CO₂ (45) permettant d'acheminer du CO₂ liquide du réservoir (40) au dispositif de compression ou de pompage (42), puis dudit dispositif de compression ou de pompage (42) à une unité de traitement (1) cryogénique,
- une unité de traitement (1) cryogénique comprenant une ou plusieurs buses (4) de distribution de jet de CO₂, alimentées en CO₂ liquide par ladite au moins une canalisation d'acheminement de CO₂ (45),
- une première unité de pulvérisation comprenant un réservoir de liquide détergent/désinfectant alimentant fluidiquement un ou plusieurs moyens de pulvérisation permettant de pulvériser au moins une partie du détergent/désinfectant provenant dudit réservoir à liquide détergent/désinfectant, et
- une unité de brossage comprenant une ou plusieurs brosses.

9. Installation selon la revendication 8, **caractérisée en ce que** la première unité de pulvérisation comprend une ou plusieurs buses de pulvérisation.

10. Installation selon la revendication 8, **caractérisée en ce que** unité de brossage comprend des brosses rotatives.

11. Installation selon l'une des revendications 8 à 10, **caractérisée en ce qu'**elle comprend en outre une deuxième unité de pulvérisation comprenant un réservoir de biocide alimentant fluidiquement un générateur de nébulisât permettant de nébuliser au moins une partie du biocide provenant dudit réservoir à biocide.

12. Installation selon l'une des revendications 8 à 11, **caractérisée en ce qu'**elle comprend en outre :
- un dispositif convoyeur (12) permettant d'acheminer au moins un dispositif médical à nettoyer au sein d'au moins l'unité de traitement (1) cryogénique, et/ou
- une unité de compression d'air (46) permettant d'alimenter la ou les buses (4) avec de l'air comprimé.

13. Installation selon l'une des revendications 8 à 12, **caractérisée en ce qu'**elle comprend en outre une unité de pilotage (16) comprenant un coffret de commande (43) destiné à réguler la distribution de CO₂ liquide et d'air comprimé à ladite ou auxdites buses (4).

14. Installation selon l'une des revendications 8 à 13, **caractérisée en ce qu'**au moins une première unité de pulvérisation et/ou au moins une unité de brossage sont agencées en amont de l'unité de traitement (1) cryogénique.

15. Installation selon l'une des revendications 8 à 14, **caractérisée en ce qu'**au moins une deuxième unité de pulvérisation est agencée en aval de l'unité de traitement (1) cryogénique.
